# EUROPEAN PATENT SPECIFICATION

(11) **EP 2 588 134 B1**
(45) Date of publication and mention of the grant of the patent: **26.10.2016**
(21) Application number: 11728285.5
(22) Date of filing: 30.06.2011
(51) Int. Cl.: A61K 39/125, C07K 14/085, A61P 3/10, G01N 33/68

(54) **DIAGNOSTIC ASSAY FOR TYPE 1 DIABETES**
DIAGNOSTISCHER TEST FÜR TYP-1-DIABETES
MÉTHODE DIAGNOSTIQUE POUR LE DIABÈTE DE TYPE 1

(30) Priority: 01.07.2010 EP 10305719; 01.07.2010 EP 10305717; 01.07.2010 EP 10305714
(43) Date of publication of application: 08.05.2013
(73) Proprietor: Vactech OY, 33520 Tampere (FI)
(72) Inventor: HYÖTY, Heikki, 33230 Tampere (FI); KNIP, Mikael, 00130 Helsinki (FI); LAITINEN, Olli, 33310 Tampere (FI); TOLONEN, Outi, 90120 Oulu (FI); HANKANIEMI, Minna, 33560 Tampere (FI); OIKARINEN, Sami, 33500 Tampere (FI); HONKANEN, Hanna-Riikka, 60510 Hyllykallio (FI); LECOUTURIER, Valérie, 69380 Chazay d'Azergues (FR); ALMOND, Jeffrey, 69570 Dardilly (FR)
(74) Representative: Kolster Oy Ab
(86) International application number: PCT/EP2011/061001
(87) International publication number: WO 2012/001098

(56) References cited:
- JAÏDANE HELA ET AL: "Enteroviruses and type 1 diabetes: towards a better understanding of the relationship.", REVIEWS IN MEDICAL VIROLOGY SEP 2010 LNKD- PUBMED:20629044, vol. 20, no. 5, 21 April 2010 (2010-04-21) , pages 265-280, XP002660770, ISSN: 1099-1654
- TAURIAINEN SISKO ET AL: "Enteroviruses in the pathogenesis of type 1 diabetes", SEMINARS IN IMMUNOPATHOLOGY, vol. 33, no. 1, 28 April 2010 (2010-04-28) , pages 45-55, XP002660771, ISSN: 1863-2297
- KLEMOLA PAIVI ET AL: "Diabetogenic Effects of the Most Prevalent Enteroviruses in Finnish Sewage", ANNALS OF THE NEW YORK ACADEMY OF SCIENCES BLACKWELL PUBLISHING, 9600 GARSINGTON RD, OXFORD OX4 2DQ, OXEN, UK SERIES : ANNALS OF THE NEW YORK ACADEMY OF SCIENCES (ISSN 0077-8923(PRINT)), 2008, pages 210-212, XP002660772, & 9TH INTERNATIONAL CONGRESS OF THE IMMUNOLOGY-OF-DIABETIES-SOCIETY/AME RICAN-DIABETIES-ASSOCIATION-RES; MIAMI BEACH, FL, USA; NOVEMBER 14 18, 2007
- OIKARINEN SAMI ET AL: "Enterovirus RNA in Blood Is Linked to the Development of Type 1 Diabetes", DIABETES, AMERICAN DIABETES ASSOCIATION, US, vol. 60, no. 1, 13 October 2010 (2010-10-13), pages 276-279, XP009152847, ISSN: 0012-1797, DOI: 10.2337/DB10-0186
- TRACY S ET AL: "Toward testing the hypothesis that group B coxsackieviruses (CVB) trigger insulin-dependent diabetes: inoculating nonobese diabetic mice with CVB markedly lowers diabetes incidence.", JOURNAL OF VIROLOGY DEC 2002, vol. 76, no. 23, December 2002 (2002-12), pages 12097-12111, ISSN: 0022-538X

## Description

### Field of the Invention

The present invention relates to a diagnostic assay for type 1 diabetes (T1 D), and especially to an assay for predicting the risk of contracting the disease. It also relates to a method of monitoring the efficacy of antiviral treatments aiming at prevention of T1 D. The invention still further relates to a diagnostic kit.

### Background of the Invention

Type 1 diabetes (T1 D) is a severe disease that has become more and more frequent already at a very early age. In type 1 diabetes the beta-cells of the pancreas are destroyed, which leads to insulin deficiency. The destruction of the beta-cells is believed to be caused by an autoimmune response, which in turn is assumed to be induced by a virus infection.

The connection between enteroviruses and type 1 diabetes (T1 D) has been documented in a multitude of studies. Enteroviruses have been detected in the pancreas, blood and intestinal mucosa of patients with type 1 diabetes more frequently than in control subjects and prospective studies have supported their role in the initiation of the beta-cell damaging process associated with type 1 diabetes.

Type 1 diabetes is typically diagnosed based on clinical symptoms when the beta-cell damaged process has already begun and the disease has burst out. However, in order to prevent or reveal the disease process it is desirable to identify subjects at risk of contracting T1D at an early stage, long before the clinical symptoms.

The suggested association between enteroviruses and T1D opens up new possibilities for predicting the risk of contracting the disease. However, the problem is that the knowledge about which serotypes may cause the disease is limited and even contradictive.

The group of enteroviruses includes more than 100 different serotypes. Enterovirus infections are usually subclinical, but they may also cause various kinds of diseases. For example polioviruses, coxsackie B virus (CBV), coxsackie A virus (CAV), and echovirus (E, or Echo) as well as numbered enteroviruses are enteroviruses known to be involved in the development of a variety of diseases. The spectrum of responsible serotypes varies a lot from disease to disease, and even in one disease like type 1 diabetes the exact serotypes have not been fully and reliably identified.

Indeed, previous studies reporting association between enteroviruses and T1D were not able to discriminate between serotype or were restricted to case reports. Some studies have suggested that CBV serotypes may be important (Yoon et al, 1979 New Eng J of Med, v300, p1173; Hindersson M, et al., 2005, J Clin Virol v.33 p158; Dotta F. et al., 2007, PNAS 104: 5115). However, other enterovirus serotypes have also been sporadically reported (Cabre-ra-Rode et al., 2003, Diabetologia; Williams et al., 2006, J Clin Micro v.44 p441).

Shibasakti et al., 2010. Endocrine Journal, Vol. 57(3):211-219 discusses the possible involvement of viral infections including i.a. CAV4-CAV6 in the pathogenesis of fulminant type 1 diabetes, but no direct evidence for the pathogenic role of the viruses in beta-cell destruction is found. Fulminant type 1 diabetes differs from classic type 1 diabetes in that it is not associated with an autoimmune T cell response, but with a virus-induced macrophage-dominated inflammatory process.

WO01/00236 (Hyöty et al.) found the following non-polio enteroviruses in children diagnosed with type 1 diabetes: CBV serotypes 1, 2, 3, 4, 5 and 6, echovirus serotypes 3, 4, 6, 9, 11, 22 and 30, CAV serotypes 9 and 16. However, no data is given as to the role of the individual serotypes listed, except for some animal tests with CBV3. A more recent publication US2010/0047273 (Rappuoli et al.) found a particular association between CBV4 and type 1 diabetes.

Filippi et al., 2008. Diabetes 57:2863-2871 is a review on the role of enteroviruses in type 1 diabetes. The results obtained in NOD mice demonstrating a link between CBV4 and diabetes or no link between CBV3 and diabetes are discussed. It is expected that there might be a fundamental difference between rodents and humans in this respect. The association between viruses and diabetes is said to be extremely complex based on mouse studies. Certain viruses are believed to be capable of both inducing and preventing diabetes in the same mouse model depending on the time of infection.

Hyöty, 2009, J. Med. Virol. 81:197-198 gives an overview of the association between enteroviruses and type 1 diabetes. The risk effect of enteroviruses was not confirmed in all prospective studies.

Jaidane et al., Reviews in Medical Virology, Sep 2010, vol. 20, no. 5, 21 April 2010, pages 265-280 is a review on the relationship between enteroviruses and T1 D.

Oikarinen et al., Diabetes, vol. 60, no. 1, October 2010, pages 276-279 discloses the detection of enterovirus RNA in blood for risk assessment of T1 D in children and concludes that enteroviruses may play a role in the initiation of the beta-cell damaging process.

For the time being there is no consensus in the prior art as to the impact of enterovirus serotypes on the development of type 1 diabetes. Still there is a great need for developing effective means and methods for predicting the risk of contracting type 1 diabetes, and diagnosing the disease at an early stage. There is also a need for monitoring the effect of antiviral treatments used for preventing the type 1 diabetes. The present invention meets these needs.

### Summary of the Invention

The present invention resides in the surprising finding that particular enterovirus serotypes are significantly more diabetogenic than others, and that in addition there are serotypes that have an opposite i.e. a highly protective effect on type 1 diabetes.

Induction of heterotypic virus resistance has previously been reported by Landau B J et al., Microbial Pathogenesis 1990:8 289-298, but not in connection with diabetes. Said publication discloses an increased resistance against liver, heart and pancreas damage caused by CBV1 infection in mice vaccinated with CBV3. CBV3 was found to cause subtotal pancreatic acinar cell damage, while CBV1 caused total acinar cell necrosis. When CBV3 vaccinated mice were challenged with CBV1 no augmentation of existing pancreatic acinar cell damage occurred. Noteworthy, however, was that neither CBV3 nor CBV1 was found to affect the insulin producing islet cells i.e. beta-cells of the pancreas.

The present inventors found that children who had been in contact with e.g. CBV3 or CBV6 had a reduced risk of contracting type 1 diabetes, while others who had been in contact with e.g. CBV1 or CBV2 had an increased risk. CBV1 was found to be significantly more strongly associated with the risk of contracting type 1 diabetes than any other enterovirus serotype. For the first time the association between infection by individual enterovirus serotypes and T1D occurrence has been demonstrated in humans, on a large case-control prospective study. It is striking that among all the serotypes studied the ones which showed the most marked risk or protective effect belonged to the CBV group. This is even more striking when the low number of CBV serotypes (six) is taken into account.

The finding of the division of enteroviruses into protective and risk serotypes opens new diagnostic implications in predicting type 1 diabetes.

The present invention provides a diagnostic assay for assessing the risk of type 1 diabetes (T1 D) comprising determining the presence of at least one risk or at least one protective enterovirus serotype in a body sample, whereby
the presence of at least one risk serotype selected from the group consisting of CBV1, CBV2, Echo3, Echo14, Echo18, Echo29, Echo32, CAV4, CAV5, CAV6, and CAV16 indicates an increased risk of contracting T1D, or
the presence of at least one protective serotype selected from the group consisting of CBV3, CBV4, CBV5, CBV6, Echo11, and Echo13 indicates a reduced risk of contracting T1 D.

The present invention further provides a method of monitoring the efficacy of antiviral treatments aiming at prevention of T1D, comprising providing a sample from a subject having received antiviral treatment, and determining the presence of at least one risk or at least one protective enterovirus serotype in the sample, whereby
the presence of at least one risk serotype selected from the group consisting of CBV1, CBV2, Echo3, Echo14, Echo18, Echo29, Echo32, CAV4, CAV5, CAV6, and CAV16 indicates that the treatment has not reduced the risk of contracting T1D, or
the presence of at least one protective serotype selected from the group consisting of CBV3, CBV4, CBV5, CBV6, Echo11, and Echo13 indicates that the treatment has reduced the risk of contracting T1 D.

Still further the invention provides the *in vitro* use of a diagnostic kit wherein the kit comprises means for determining the presence of at least one risk and at least one protective enterovirus serotype in a body sample, whereby the risk serotype is selected from the group consisting of CBV1, CBV2, Echo3, Echo14, Echo18, Echo29, Echo32, CAV4, CAV5, CAV6, and CAV16, and the protective serotype is selected from the group consisting of CBV3, CBV4, CBV5, CBV6, Echo11, and Echo13.

Some preferred embodiments of the invention are set forth in the dependent claims. Other objects, details and advantages of the present invention will become apparent from the following drawings, detailed description and examples.

### Brief Description of the Drawings

Figure 1 shows conditional logistic regression analysis for 41 viruses at autoantibody seroconversion date.
Figure 2 shows a summary of the tight and loose criteria of CBV1, CBV3 and CBV6 time window analyses.
Figure 3 shows a combined population attributable risk (PAR) for CBV1 and CBV2.
Figure 4 shows protective PAR% for CBV3 and CBV6 using classes positive vs. negative (0-3 vs. 4).
Figure 5 shows the duration of virus excretion.
Figure 6 shows the ratio of enterovirus infection and samples in age classes.
Figure 7 shows the ratio of all enterovirus infections and samples (upper panel), and infections and samples before the appearance of autoantibodies (AAB) (lower panel).

### Detailed Description of the Invention

If not otherwise indicated "type 1 diabetes" or "T1D" as used herein refers to the classic form of the disease, which is associated with the appearance of autoantibodies against pancreatic beta-cells. This disease may also be called "classic type 1 diabetes" to be distinguished from "fulminant type 1 diabetes", which is a rare form of diabetes that is associated with a macro-phage dominated inflammatory process, which does not involve autoimmune antibodies.

Typical risk groups for contracting T1 D are children with diabetes in first-degree relatives, children testing positive for diabetes-related autoantibodies, and children with genetic risk alleles for the disease.

The diagnostic assay of the invention comprises determining the presence of at least one risk and/or protective enterovirus serotype. Risk serotypes as used in this connection may also be called diabetogenic serotypes. The risk enterovirus group consists of serotypes CBV1, CBV2, Echo3, Echo14, Echo18, Echo29, Echo32, CAV4, CAV5, CAV6, and CAV16. The assay may comprise the determination of any combination of said risk viruses. The presence of CBV1 and/or CBV2 is especially indicative of an increased risk. According to one embodiment of the invention the presence of at least CBV1, or of CBV1 and CBV2 is determined. According to another embodiment of the invention at least one of CBV1, CBV2, CVB3, CVB4, CVB5 and CBV6 is determined. According to still another embodiment the presence of at least three of CBV1, CBV2, Echo32, Echo29, and Echo14 is determined, or all said five serotypes are determined. According to one further embodiment all eleven risk serotypes are determined.

The protective enterovirus group consists of serotypes CBV3, CBV4, CBV5, CBV6, Echo11 and Echo13. The assay may comprise the determination of any combination of said protective viruses. The presence of CBV3 and/or CBV6 is especially indicative of a reduced risk of T1D. Contrary, the absence of a protective serotype may also indicate an increased risk of contracting T1 D. According to one embodiment of the invention the presence of at least CBV3 and/or CBV6 is determined. According to another embodiment the presence of all six protective enteroviruses is determined.

In a preferred embodiment of the invention the presence of at least one risk enterovirus and at least one protective enterovirus is determined. Typically the presence of at least CBV1, CBV2, CBV3 and CBV6 is determined. According to one embodiment the presence of at least CBV1, CBV2, Echo32, Echo29, Echo14, CBV3, CBV6, Echo11 and Echo13 is determined. Any other combination of risk and protective viruses is also possible. In one embodiment the presence of all twelve risk serotypes and all four protective serotypes are determined.

The diagnostic assay for assessing the "risk of type 1 diabetes" may be used for predicting the risk of contracting T1D or a beta-cell damaging autoimmune process, for diagnosing T1D or a beta-cell damaging autoimmune process in a patient, or for the prognosis of the disease. Thus the presence of a risk enterovirus indicates an increased risk of contracting T1D, or an already ongoing T1D, or a bad prognosis of the disease, while the presence of a protective enterovirus indicates a reduced risk of contracting T1D, or a good prognosis of the disease, especially if the autoimmune process has not started yet, or if the autoimmune process has not proceeded to clinical T1 D. Even when the diagnosis has been made, the prognosis in the sense of severity of the disease can be better. The presence of at least one risk serotype together with at least one protective serotype, indicates a smaller risk or better prognosis for T1D than the presence of at least one risk serotype in the absence of any protective serotype.

The time and order of infection also has an influence on the risk or prognosis. The diagnostic assay may be carried out on samples taken at different times, whereby the presence of a protective serotype prior to the presence of a risk serotype in a subject indicates a smaller risk of T1D than the opposite order.

The diagnostic assay may be carried out on body samples from very young subjects, such as on a cord blood sample of a newborn, or on other types of samples from infants from the age of about 12 weeks, or earlier. According to one embodiment the assay is carried out on samples from children at the age of about 6, 12, 18 and 24 months. The assay may be carried out as a screening method on samples of a whole cohort, or from a subpopulation carrying increased risk for presenting with type 1 diabetes. Such high-risk groups may include mothers or children with HLA-conferred genetic risk (HLA DR3 and/or DR4), subjects with type 1 diabetes in first- or second-degree relatives or subjects testing positive for two or more diabetes-associated autoantibodies. Typically the body sample is from a subject suspected to develop T1D or a beta-cell damaging autoimmune process.

The diagnostic assay comprises determining the presence of particular enterovirus serotypes in a body sample. "Determining the presence" as used in this connection encompasses both direct and indirect means of showing an ongoing or past infection. Conveniently the presence of the enterovirus is determined by detecting a component thereof, or an immune response thereto. The component may be a structure of said virus e.g. a nucleic acid or an encoded polypeptide, such as VP1, VP2, VP3, VP4, 2A, 2B, 2C, 3A, 3B, 3C or 3D, or parts thereof. The immune response may be measured by determining an antibody or a T-cell specifically recognizing the viral polypeptide. The assay may be used to identify a persistent or acute infection by risk or protective serotypes. The determination of an immune response may also reveal past infections in the subject. The virus may also be detected by cultivation.

The body sample to be tested can be any sample from the body, wherein the virus or immune response is present, such as a blood, saliva, stool or tissue sample. The presence of the virus may be determined e.g. in a stool sample by PCR, or antibodies against the virus may be determined in a blood sample taken from the subject to be tested e.g. by a plaque inhibition assay or microneutralizaton analysis of the serum or plasma sample as described in the examples. In addition, the virus may be detected in various tissues using e.g. RT-PCR, *in situ* hybridization or immunohistochemistry.

According to one embodiment, the presence of the virus is determined by PCR, RT-PCR, *in situ* hybridization, immunohistochemistry, antigen detection using EIA or other methods, virus isolation, sequencing, electron microscopy, or immuno electron microscopy. According to another embodiment the presence of the virus is determined by detecting an immune response against the virus by measuring virus specific antibody response e.g. by neutralization assay or other antibody assays, or by measuring cell-mediated immune response to the virus e.g. using T-cell proliferation assays or other methods measuring cell-mediated immunity, or measurements of innate immune system activation.

The assay may be used to detect a high or low risk profile of antibodies in serum, blood, saliva or stools using seroneutralization against risk and protective serotypes. It may also be used to detect virus structures specific for high risk and protective serotypes in tissue and blood. It may further be used to detect viral genome of risk or protective serotypes in *in situ* hybridization with specific probes. The assay may still further be used in PCR, which specifically amplifies the genome of risk or protective viruses, or in hybridization with oligonucleotide probes to detect PCR amplicons specific for the risk and protective serotypes. Other methods encompass sequencing of a protein sequence from tissues to identify the risk and protective serotypes, or virus isolation to detect the risk and protective serotypes.

"Seroneutralization" is an analysis, in which the ability of given virus-specific antibodies possibly present in a body sample to inhibit the multiplication of the given virus is determined. It can be measured as plaque number reduction compared to a control sample in cell culture. Enteroviruses form plaques in virus-sensitive cell cultures. This plaque-forming ability can be used in analyzing the enterovirus antibodies. Serotype-specific enterovirus antibodies present in e.g. serum or plasma neutralize the enteroviruses and prevent or reduce the plaque formation. In this assay, a defined dilution of study sample is mixed with equivalent volume of enterovirus of known titer, this mixture is added to cells and the presence of enterovirus antibodies is detected by reduction in the number of plaques when compared with non-neutralized virus.

The diagnostic methods for determining the presence of the risk or protective serotypes is also useful in monitoring the efficiency of antiviral treatments aiming at prevention of T1D. The antiviral treatment may aim at eradication or prevention of infections with said viruses, or at prevention of the beta-cell damaging autoimmune process. Such antiviral treatment may be e.g. treatment with capsid binders, protease inhibitors, nucleoside analogs, immunoglobulins, monoclonal antibodies or interferon alpha and beta.

The diagnostic kit for use in the diagnostic assay or method of monitoring the efficacy of antiviral treatment may comprise any reagents needed for determination of the particular enterovirus serotypes to be determined. Optionally the kit also contains instructions for use.

The present invention is illustrated by the following non-limiting examples.

### Example 1

In the present study diabetogenic serotypes were identified by measuring neutralizing antibodies in serum and by typing viruses directly from stool samples in children who were observed in a prospective birth cohort study. Serum samples were available from a large series of children while stool samples were derived from a smaller cohort. Thus, the main data in this study is based on serum analyses while stool samples provided information on circulating enteroviruses and allowed their isolation for neutralizing antibody analyses.

### Seroneutralization analyses

Neutralizing antibodies were analyzed against a wide panel of different enterovirus serotypes in the same serum sample which was the first autoantibody positive sample taken during the prospective follow-up. Thus, this time point represents the initiation of the beta-cell damaging process. In addition, antibodies against those serotypes which were found to be interesting in this initial screening were measured at additional time-points to study the timing of infections and their relationship with the initiation of the beta-cell damaging process.

Altogether, seroneutralization analyses were performed using 44 viruses and 522 serum/plasma samples (174 triplets, two control children for each case child). In order to study the effect of the strain variation, two serotypes (CBV4 and Echo3) have been analyzed using both the freshly isolated wild type strains (wt) and corresponding reference (ATCC) strains (rs).

The viruses were analyzed in seroneutralization analyses using autoantibody seroconversion date samples. The viruses were CAV4, CAV5, CAV10, CAV16, EV71, CAV9, CBV1, CBV2, CBV3, CBV4-wt, CBV4-rs, CBV5, CBV6, Echo1, Echo2, Echo3-wt, Echo3-rs, Echo4, Echo5, Echo6, Echo7, Echo9, Echo11, Echo12, Echo13, Echo14, Echo15, Echo17, Echo18, Echo19, Echo20, Echo21, Echo25, Echo26, Echo27, Echo29, Echo30, Echo32, Echo33, EV74, EV78 and EV94.

The seroneutralization analyses were carried out using a plaque neutralization assay (Roivainen M, Knip M, Hyöty H, Kulmala P, Hiltunen M, Vähäsalo P, Hovi T, A Akerblom HK. 1998. Several different enterovirus serotypes can be associated with prediabetic autoimmune episodes and onset of overt IDDM. Childhood Diabetes in Finland (DiMe) Study Group. J Med Virol 56:74-78.). In this analysis the ability of the serum/plasma to inhibit a certain virus's ability to form plaques in cell layers was determined as compared to controls, in which fetal calf serum has been used instead of human serum. In the analysis the inhibition has been considered to be significant (positive result, ++) when it has been more than 85%. The inhibition range between 85-75% has been considered as weaker positive (+) and inhibition of less than 75% has been judged as a negative result. Because these analyses have been performed using two different serum/plasma dilutions (1/4 and 1/16) the results have been combined using the classification shown below.

| **Class** | **1:4** | **1:16** |
|---|---|---|
| Max positive (0) | ++ | ++ |
| Highly positive (1) | ++ | + |
| Moderately positive (2) | ++ | - |
| Positive (3) | + | - |
| Negative (4) | - | - |

### Seroneutralization results

The raw data was exported to Stata package and analyzed using conditional logistic regression models to evaluate the risk of certain serotypes to cause T1 D. The results of the conditional logistic regression analysis are given as odds ratios (ORs). If the OR in certain analysis is higher than 1 and the lower limit of the confidence interval (Cl at the level of 95%) remains above 1 the virus can be considered as conferring risk for T1 D. On the other hand, if both the OR and the higher limit of the 95% Cl are below 1, such a serotype can be considered as protective against T1 D. In such cases where OR is either over or below 1 and also the 95% Cl includes values on both side of 1, the result is not statistically significant (P>0.05). In Table 1 the OR and Cl values for the most inter-estinq viruses are presented.

**Table 1. The OR and Cl values of the most interesting serotypes. The statistically significant results are bolded.**

| Virus | [OR (Cl)] | [OR (Cl)] | [OR (Cl)] | |
|---|---|---|---|---|
| | 0-3 vs. 4 | 0-1 vs. 2-4 | 0-2 vs. 4 | 0-1 vs. 4 |
| CBV1 | **1.50 (1.02-2.23)** | 1.10 (0.65-1.87) | 1.56 (0.94-2.58) | 1.39 (0.68-2.85) |
| CBV3 | **0.39 (0.18-0.82)** | 0.56 (0.24-1.34) | **0.36 (0.15-0.85)** | 0.50 (021-1.20) |
| CBV6 | **0.64 (0.41-0.97)** | 0.57 (0.21-1.59) | 0.86 (050-1.51) | 049 (0.16-1.57) |

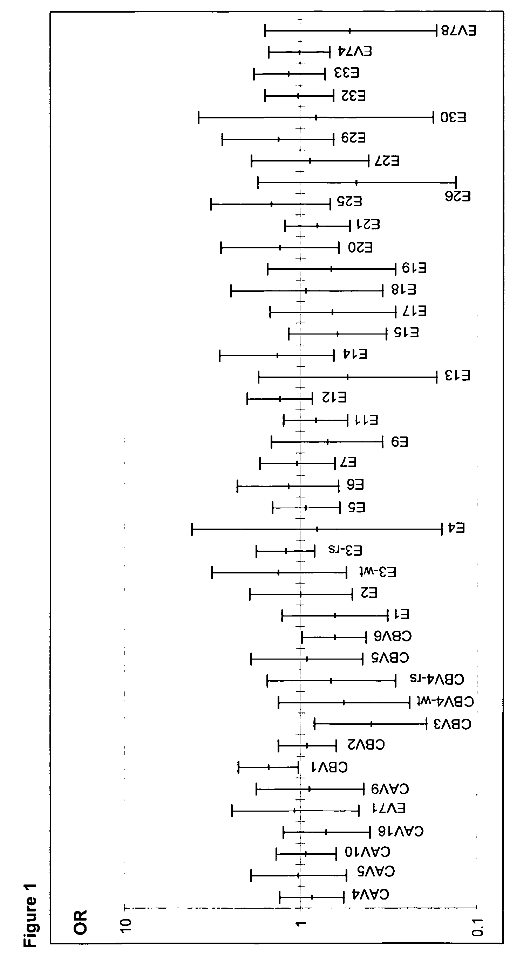

The results for the tested viruses are shown in Figure 1 for whole cohort data, classes 0-3 vs. 4.

The prevalence of antibodies (percentage of children having neutralizing antibodies against each virus serotype) was also calculated.

It was found that especially CBV3 and CBV6 are protective serotypes, while especially CBV1 is a clear risk serotype for type 1 diabetes. These associations were first drawn from seroneutralization results of a single cross-sectional time-point representing the first autoantibody positive sample, and were later confirmed in multi-time point analyses in which the timing of infections was analyzed in more detail. In these multi-time-point analyses the clearest result was the strong association of the CBV1 risk effect to the six month time window immediately preceding the first detection of type 1 diabetes related autoantibodies. Other CBV serotypes than CBV1, CBV3 and CBV6 did not show such a clear risk or protective effect in the single time-point analysis carried out at autoantibody seroconversion.

### Multi-time point seroneutralization analysis with CBV1, CBV3 and CBV6

To clarify the possible order and timing of CBV1, CBV3 and CBV6 infections, the following time points were selected for the seroneutralization analyses:
- 12 months before autoantibody seroconversion
- 6 months before autoantibody seroconversion
- point (first detection of autoantibodies; these samples were analyzed previously)
- 12 months after autoantibody seroconversion
- time of the diagnosis of type 1 diabetes

According to this plan approximately 1250 new samples fulfilling the criteria above were identified, collected, anonymized and tested.

All samples were screened using 1/4 and 1/16 serum dilutions. In this report the class comparison 03- vs. 4 and sensitivity analyses 1 and 2 has been generally utilized, except in the tight criteria analyses (explained below) in which infections were diagnosed by subjective judgments done by two independent researchers on the basis of pre-fixed criteria listed below for acute infection:
The acute infection was diagnosed according to following criteria which had to be true for a classified infection:
   - A seroconversion from titer 0 to titer 4 or higher
   - The titer is 16 in at least one of the following samples
   - All follow-up samples remain positive

All analyses were done blindly without knowing the case-control status of the child.

### Temporarily peaking antibodies and maternal antibodies

The accumulated data from new time points concerning CBV1, CBV3 and CBV6 clearly showed that the previously analyzed 1/4 and 1/16 dilutions at the time of seroconversion to autoantibody positivity do not provide the whole picture of the infection histories of all children. When these new time points data are taken into account, transient increases in antibody titers were seen against all of these three serotypes either separately or in different combinations in many children. These transient rises may have different explanations: In some cases they are clearly remnants of maternal antibodies. In other cases the reason may be antibody cross-reactivity between closely related serotypes. This issue has been studied more thoroughly in a separate sub-study. In addition, it is possible that the virus strain used in the neutralizing assay is antigenically different from the one causing the infection, and then it may not detect low antibody titers. Furthermore, we cannot rule out the unlikely possibility that some samples may also contain other substances than antibodies that may inhibit virus growth. Finally, there is always a possibility of variation in the assay performance due to technical reasons.

How to deal with this quandary? We have approached this concern using a three-sided strategy:
1. minimizing the influence of wrong negatives = loose criteria
2. minimizing the influence of wrong positives = tight criteria
3. removing the effect of maternal antibodies, otherwise like loose criteria = middle criteria

The loose criteria approach means that basically all results from different time points have been accepted and analyzed as they are. In the middle criteria approach all other results have been accepted except those that have possibly been biased by maternal antibodies. In the tight criteria approach we have carefully gone through the data and excerpted only those infections which fulfill previously set criteria of acute infection.

### Multi-time point statistical analyses

All statistical analyses presented are based on conditional logistic regression analyses. Three types of analyses were carried out for this data set. 1) The timing of infections was determined and the time-relationship between infections and appearance of autoantibodies was analyzed. In other words, the frequency of infections among case children were compared to that in control children in each time window separately. 2) The mutual order of CBV1, CBV3 and CBV6 infections was analyzed separately in case and control children and the interactions between the three serotypes were also analyzed. 3) The new time point results were analyzed similarly as described above by comparing cases and controls in each time point separately. These analyses were carried out in the whole group and in different subgroups according to the following list:
- Total data set from the whole cohort
- Gender
- Age
- HLA genotype
- Residence area
- Combinations of different AABs in certain follow-up samples
- Diagnosis of diabetes
- Cumulative protective effect

Seroneutralization analyses of these three viruses have been carried out using a plaque neutralization assay as described above.

Statistical analyses in loose and middle criteria approaches were done according to combined classes 0-3 vs. 4 and also sensitivity analyses, in which class 3 (sensitivity analysis 1) or both classes 2 and 3 (sensitivity analysis 2) are removed from the data set, were utilized. In the tight criteria approach the sensitivity analyses were not done due to its intrinsic "sensitivity" character.

Antibodies to islet cells were detected by indirect immunofluorescence, and antibodies to insulin, glutamic acid decarboxylase, and protein tyrosine phosphatase-like protein (IA-2) were determined with specific radiobinding assays from a serum sample using standard methods. If two of these four autoantibodies were detected, the subject was considered autoantibody positive (Näntö-Salonen et al., 2008. Lancet 372:1746-1755).

### Timing of the infections

The timing of infections caused by the three CBV serotypes of interest was approached in the following way: First, the recognized infections were categorized to different time windows relative to the date of seroconversion to autoantibody positivity (AAB+ date). The used windows were as follows:
0. No infections or infection after the AAB+ date
1. Infection more than 12 months before the AAB+ date
2. Infection between 12 and 6 months before the AAB+ date
3. Infection within 6 months before the AAB+ date

In addition to using these time-windows separately, analyses were also performed by combining some of these windows.

In the loose criteria approach the first positive result was accepted as an infection regardless of the possibility of maternal antibodies or the possibility that results of the later time point samples turn to negative. The middle criteria approach was done similarly, except that those results that were biased by the identified maternal antibodies were nullified. This judgement was done by two independent experts who evaluated the data carefully and discarded those results in which the maternal antibodies could be the cause of the positivity. In the tight criteria approach the similar judgement procedure were applied. In short the acute infection according to tight criteria were as follows:
- a seroconversion from titer 0 to titer 4 or higher
- the titer is 16 in at least one of the following samples
- all follow-up samples remain positive

In addition to using these time-windows separately, analyses were also performed by combining some of these windows together.

The timing of infections caused by the CBV1 risk serotype in these time windows is summarized in Tables 2 and 3. These analyses were done using the loose criteria and middle criteria and the risk effect of infection was analyzed by comparing its frequency in different time-windows to that in window zero (see above). The basic statistical analyses i.e. classes 0-3 vs. 4. and sensitivity analyses 1 and 2 were done.

**Table 2. CBV1 timing conditional logistic regression analysis, other windows vs. window 0. Loose criteria approach with classes 0-3 vs. 4 and sensitivity analyses 1 and 2.**

| **CBV1** | **Odds ratio** | **P-value** | **95% Conf. Interval** | |
|---|---|---|---|---|
| **Class 0-3 vs. 4** | | | | |
| Over 12 months before AAB+ date | 1.77 | 0.064 | 0.97 | 3.25 |
| 12-6 months before AAB+ date | **2.24** | **0.007** | **1.25** | **4.00** |
| 6-0 months before AAB+ date | **3.56** | **< 0.001** | **1.92** | **6.62** |

| **Sensitivity 1, Class 0-2 vs. 4** | | | | |
|---|---|---|---|---|
| Over 12 months before AAB+ date | 0.89 | 0.724 | 0.46 | 1.72 |
| 12-6 months before AAB+ date | 1.25 | 0.468 | 0.68 | 2.31 |
| 6-0 months before AAB+ date | **2.19** | **0.018** | **1.15** | **4.17** |

| **Sensitivity 2, Class 0-1 vs. 4** | | | | |
|---|---|---|---|---|
| Over 12 months before AAB+ date | 1.19 | 0.684 | 0.51 | 2.79 |
| 12-6 months before AAB+ date | 0.98 | 0.950 | 0.47 | 2.03 |
| 6-0 months before AAB+ date | 2.09 | 0.067 | 0.95 | 4.59 |

**Table 3. CBV1 timing conditional logistic regression analysis, other windows vs. window 0. Middle criteria approach with classes 0-3 vs. 4 and sensitivity analyses 1 and 2.**

| **CBV1** | **Odds ratio** | **P-value** | **95% Conf. Interval** | |
|---|---|---|---|---|
| **Class 0-3 vs. 4** | | | | |
| Over 12 months before AAB+ date | 1.62 | 0.262 | 0.70 | 3.75 |
| 12-6 months before AAB+ date | 0.77 | 0.417 | 0.41 | 1.44 |
| 6-0 months before AAB+ date | **2.09** | **0.002** | **1.32** | **3.33** |

| **Sensitivity 1, Class 0-2 vs. 4** | | | | |
|---|---|---|---|---|
| Over 12 months before AAB+ date | 1.14 | 0.795 | 0.41 | 3.17 |
| 12-6 months before AAB+ date | 0.66 | 0.307 | 0.30 | 1.46 |
| 6-0 months before AAB+ date | **2.10** | **0.007** | **1.23** | **3.57** |

| **Sensitivity 2, Class 0-1 vs. 4** | | | | |
|---|---|---|---|---|
| Over 12 months before AAB+ date | 0.70 | 0.629 | 0.16 | 3.02 |
| 12-6 months before AAB+ date | 0.36 | 0.078 | 0.11 | 1.12 |
| 6-0 months before AAB+ date | 1.44 | 0.342 | 0.68 | 3.05 |

Interestingly, in these analyses the CBV1 infections are clearly found more often in case children according to the risk hypothesis with infections occurring close to the time of autoantibody seroconversion (at the time window 6-0 months before the AAB+ date). The result is the same in all analyses, except that in sensitivity analysis 2 the statistical significance is missed. Because in this analysis we have accepted all positive antibody results (probably including false positive findings), except maternal antibodies in middle criteria analyses, it is essential to compare these results to those obtained using tight criteria for infections (Table 4).

**Table 4. CBV1 timing conditional logistic regression analysis, other windows vs. window 0. Tight criteria approach.**

| **CBV1** | **Odds ratio** | **P-value** | **95% Conf. Interval** | |
|---|---|---|---|---|
| Over 12 months before AAB+ date | 0.67 | 0.455 | 0.24 | 1.89 |
| 12-6 months before AAB+ date | 1.38 | 0.491 | 0.55 | 3.46 |
| 6-0 months before AAB+ date | **3.76** | **0.001** | **1.68** | **8.44** |

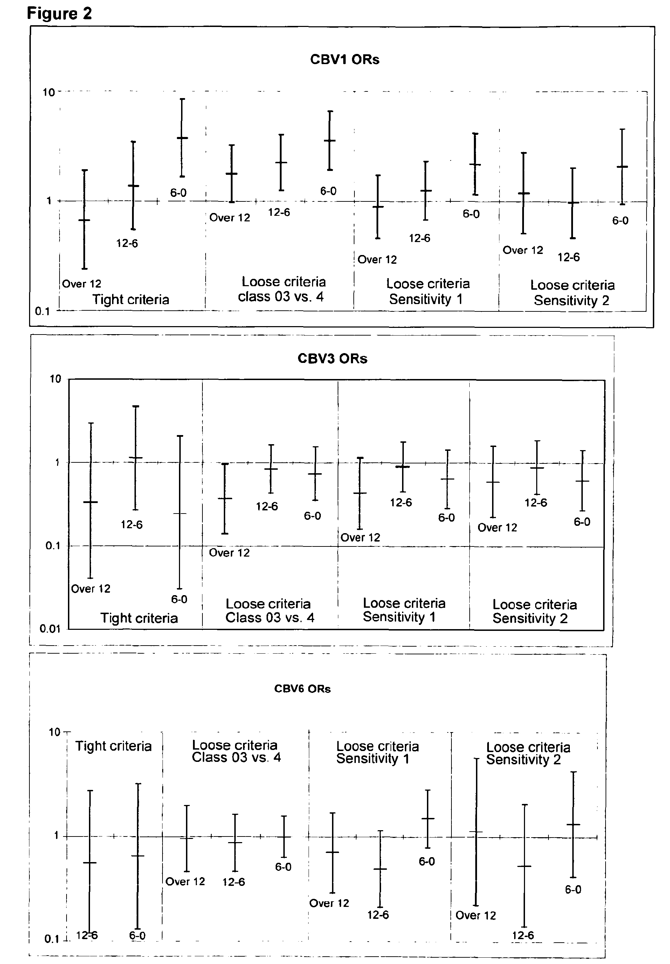

In this analysis, a lot of data have been removed to exclude all possible wrong positives. The results show that a credible dose response curve is observed highlighting the importance of the time window 6-0 months before the AAB+ date and proving a definite risk effect of CBV1 with convincing statistical outcome. Most importantly, because the same 6-0 months before the AAB+ date window was observed to be statistically significant in both the loose, middle and tight criteria approaches, these results cement this time period as a critical one for the CBV1 infection as a risk to induce T1 D. The tight criteria and loose criteria results are presented together in Figure 2, which shows CBV1, CBV3 and CBV6 ORs in different time windows before AAB+ date, Left: tight criteria, Right: loose criteria (class 0-3 vs. 4, sensitivity 1, sensitivity 2). The dose response curves can be seen with both approaches being the most conspicuous in the tight criteria and loose criteria sensitivity 1 analyses.

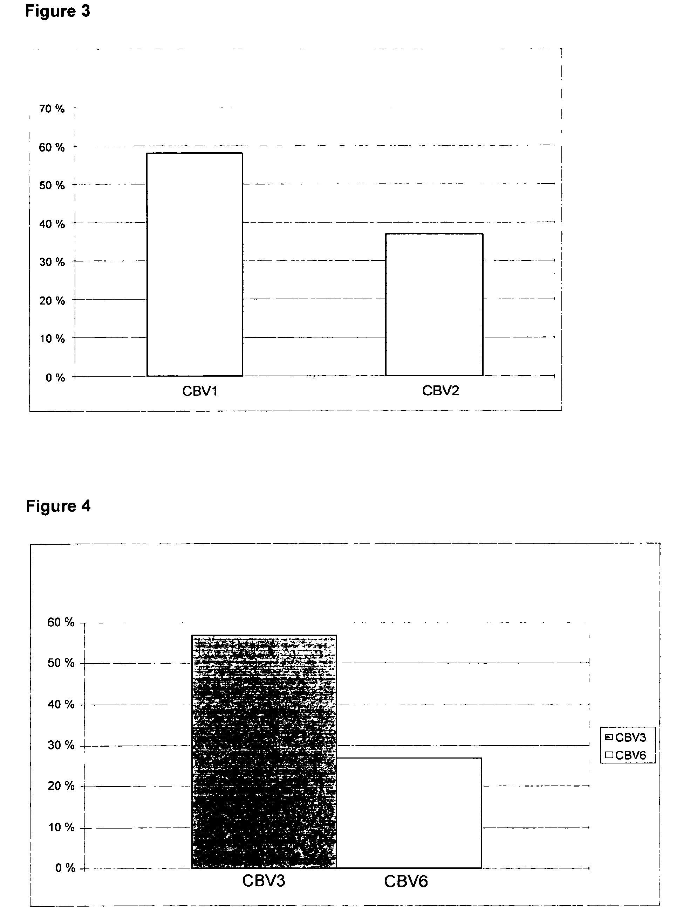

In order to understand the significance of this result let us interpret it in the terms of the population attributable risk (PAR) which estimates the proportion of type 1 diabetes cases which could be prevented by the CBV1 vaccine in the population. Assuming that the OR is 3.76 (= tight criteria result for window 6-0 months before the AAB+ date) and the prevalence of the CBV1 infection is 50% as indicated by the prevalence of CBV1 antibodies in AAB+ time point in control subjects, the PAR equation results in 58% for CBV1 alone (Figure 3). Figure 3 shows the population attributable risk (PAR) for CBV1 calculated using an OR of 3.76 and a prevalence (Pc) of 50% representing the seroprevalence at the time of autoantibody seroconversion in control subjects.

In the following analysis the time windows 6-0 and 12-6 were combined. The risk effect of CBV1 was significant also in this combined time window analysis (Table 5).

**Table 5. CBV1 timing conditional logistic regression analysis, 12-0 window against window 0. Tight criteria approach.**

| **CBV1** | **Odds ratio** | **P-value** | **95% Conf. Interval** | |
|---|---|---|---|---|
| Over 12 months before AAB+ date | 0.66 | 0.434 | 0.24 | 1.85 |
| 12-0 months before AAB+ date | **2.46** | **0.004** | **1.33** | **4.53** |

Because the strongest risk effect was seen in the 6-0 window, this time period was analyzed using another type of comparison. Instead of comparing it against window 0 (which was done in the previous analyses), this window was compared to all other windows in combination. Again, the previous findings showing the critical importance of this time-window were supported by this analysis (Table 6).

**Table 6. CBV1 timing conditional logistic regression analysis, 6-0 window against combined other time windows. Tight criteria approach.**

| **CBV1** | **Odds ratio** | **P-value** | **95% Conf. Interval** | |
|---|---|---|---|---|
| 6-0 months before AAB+ date | **3.78** | **0.001** | **1.69** | **8.43** |

Similar analyses were performed for the protective serotypes CBV3 and CBV6. Tables 7, 8, 9 and 10 present the results of different time points for CBV3 and CBV6, respectively, when analyzed using loose and middle criteria. The tight criteria and loose criteria results are presented in Figure 2 for both CBV3 and CBV6. Protection by CBV3 was seen when infection occurred early. The effect was weakened when maternal antibodies were excluded (middle criteria) suggesting that maternal antibodies may provide part of this protection.

**Table 7. CBV3 timing conditional logistic regression analysis, other windows against window 0. Loose criteria approach.**

| **CBV3** | **Odds ratio** | **P-value** | **95% Conf. Interval** | |
|---|---|---|---|---|
| **Class 0-3 vs. 4** | | | | |
| Over 12 months before AAB+ date | **0.37** | **0.038** | **0.14** | **0.95** |
| 12-6 months before AAB+ date | 0.84 | 0.618 | 0.43 | 1.64 |
| 6-0 months before AAB+ date | 0.74 | 0.439 | 0.35 | 1.57 |

| **Sensitivity 1** | | | | |
|---|---|---|---|---|
| Over 12 months before AAB+ date | 0.43 | 0.086 | 0.16 | 1.13 |
| 12-6 months before AAB+ date | 0.90 | 0.754 | 0.45 | 1.77 |
| 6-0 months before AAB+ date | 0.64 | 0.280 | 0.28 | 1.44 |

| **Sensitivity 2** | | | | |
|---|---|---|---|---|
| Over 12 months before AAB+ date | 0.59 | 0.291 | 0.22 | 1.58 |
| 12-6 months before AAB+ date | 0.87 | 0.724 | 0.42 | 1.84 |
| 6-0 months before AAB+ date | 0.62 | 0.266 | 0.27 | 1.44 |

**Table 8. CBV6 timing conditional logistic regression analysis, other windows against window 0. Loose criteria approach.**

| **CBV6** | **Odds ratio** | **P-value** | **95% Conf. Interval** | |
|---|---|---|---|---|
| **Class 0-3 vs. 4** | | | | |
| Over 12 months before AAB+ date | 0.92 | 0.772 | 0.53 | 1.61 |
| 12-6 months before AAB+ date | 1.53 | 0.098 | 0.92 | 2.52 |
| 6-0 months before AAB+ date | 1.33 | 0.264 | 0.80 | 2.22 |

| **Sensitivity 1** | | | | |
|---|---|---|---|---|
| Over 12 months before AAB+ date | 0.37 | 0.060 | 0.13 | 1.04 |
| 12-6 months before AAB+ date | 0.74 | 0.410 | 0.37 | 1.51 |
| 6-0 months before AAB+ date | 1.04 | 0.912 | 0.52 | 2.08 |

| **Sensitivity 2** | | | | |
|---|---|---|---|---|
| Over 12 months before AAB+ date | 0.26 | 0.227 | 0.03 | 2.33 |
| 12-6 months before AAB+ date | 0.57 | 0.354 | 0.17 | 1.88 |
| 6-0 months before AAB+ date | 0.90 | 0.888 | 0.20 | 3.98 |

**Table 9. CBV3 timing conditional logistic regression analysis, other windows against window 0. Middle criteria approach.**

| **CBV3** | **Odds ratio** | **P-value** | **95% Conf. Interval** | |
|---|---|---|---|---|
| **Class 0-3 vs. 4** | | | | |
| Over 12 months before AAB+ date | 0.50 | 0.535 | 0.06 | 4.47 |
| 12-6 months before AAB+ date | 0.61 | 0.557 | 0.11 | 3.22 |
| 6-0 months before AAB+ date | 0.62 | 0.327 | 0.24 | 1.60 |

| **Sensitivity 1** | | | | |
|---|---|---|---|---|
| Over 12 months before AAB+ date | 0.50 | 0.535 | 0.06 | 4.47 |
| 12-6 months before AAB+ date | 0.61 | 0.557 | 0.11 | 3.22 |
| 6-0 months before AAB+ date | 0.42 | 0.147 | 0.13 | 1.36 |

| **Sensitivity 2** | | | | |
|---|---|---|---|---|
| Over 12 months before AAB+ date | 0.50 | 0.535 | 0.06 | 4.47 |
| 12-6 months before AAB+ date | 0.39 | 0.405 | 0.04 | 3.59 |
| 6-0 months before AAB+ date | 0.47 | 0.213 | 0.14 | 1.54 |

**Table 10. CBV6 timing conditional logistic regression analysis, other windows against window 0. Middle criteria approach.**

| **CBV6** | **Odds ratio** | **P-value** | **95% Conf. Interval** | |
|---|---|---|---|---|
| **Class 0-3 vs. 4** | | | | |
| Over 12 months before AAB+ date | 0.96 | 0.902 | 0.46 | 1.97 |
| 12-6 months before AAB+ date | 0.87 | 0.653 | 0.46 | 1.62 |
| 6-0 months before AAB+ date | 0.99 | 0.969 | 0.63 | 1.56 |

| **Sensitivity 1** | | | | |
|---|---|---|---|---|
| Over 12 months before AAB+ date | 0.70 | 0.433 | 0.29 | 1.69 |
| 12-6 months before AAB+ date | 0.49 | 0.097 | 0.21 | 1.14 |
| 6-0 months before AAB+ date | 1.48 | 0.222 | 0.79 | 2.79 |

| **Sensitivity 2** | | | | |
|---|---|---|---|---|
| Over 12 months before AAB+ date | 1.12 | 0.891 | 0.22 | 5.62 |
| 12-6 months before AAB+ date | 0.53 | 0.358 | 0.14 | 2.04 |
| 6-0 months before AAB+ date | 1.34 | 0.620 | 0.42 | 4.27 |

Tight criteria results for same viruses are shown in table 11.

**Table 11. CBV3 and CBV6 timing conditional logistic regression analysis, other windows against window 0. Tight criteria approach. *There were no CBV6 infections in cases over 12 months window according to tight criteria approach. Therefore statistical results could not be calculated for that window. However, in controls infections were recognized also in this time window supporting the protective effect of CBV6.**

| **CBV3** | **Odds ratio** | **P-value** | **95% Conf. Interval** | |
|---|---|---|---|---|
| Over 12 months before AAB+ date | 0.33 | 0.326 | 0.04 | 2.95 |
| 12-6 months before AAB+ date | 1.13 | 0.870 | 0.27 | 4.74 |
| 6-0 months before AAB+ date | 0.24 | 0.195 | 0.03 | 2.09 |

| **CBV6** | | | | |
|---|---|---|---|---|
| Over 12 months before AAB+ date | -* | -* | -* | -* |
| 12-6 months before AAB+ date | 0.56 | 0.468 | 0.12 | 2.70 |
| 6-0 months before AAB+ date | 0.65 | 0.592 | 0.13 | 3.21 |

In most of these analyses for CBV3 and CBV6 a trend can be seen that the former the time window is the more protective character both these viruses show. These results as such are logical: The protective virus should precede the risk virus, assuming that the protective effect is mediated by immunological cross-protection. The same trend is seen for both viruses in most of the analyses.

As a conclusion from the timing analyses it can be seen that the critical timing of the risk virus CBV1 infection falls into the time window one year and especially 6 months before the AAB+ date. The results also supported the protective properties of CBV3 and CBV6.

If protective, the lack of said virus can be seen as a risk for T1 D. Therefore the inverse prevalence and inverse OR was used for protective PAR% calculation using positive versus negative (0-3 vs 4). The result of the protective PAR% calculations was 57% for CBV3 and 27% for CBV6, as also shown in Figure 4.

### Order of the infections - chronology

In this analysis different possibilities of the order of infections were first classified to following classes:
0 = not risk or protective viruses at any time point
1 = first infection by the risk virus, CBV1
2 = first infection by the protective virus, CBV3 or CBV6, alone or both
3 = first infection by the risk and protective virus in the same time-window

In this first analysis the classes "first risk infection" (class 1 above) and "combined protective class" (class 2) were analyzed. Statistical comparisons were made against class "not risk or protective viruses at any time-point" (class 0). Results of these analyses are summarized in Table 12 (tight criteria), Table 13 (loose criteria) and Table 14 (middle criteria). In these analyses the follow-up period till the AAB+ date has been taken into account.

**Table 12. Order of the infections (conditional logistic regression analysis), compared to class 0. Tight criteria approach.**

| **Order** | **Odds ratio** | **P-value** | **95% Conf. Interval** | |
|---|---|---|---|---|
| First risk | 1.29 | 0.290 | 0.81 | 2.06 |
| First protective | **0.58** | **0.098** | **0.31** | **1.10** |

**Table 13. Order of the infections (conditional logistic regression analysis), compared to class 0. Loose criteria approach.**

| **Order** | **Odds ratio** | **P-value** | **95% Conf. Interval** | |
|---|---|---|---|---|
| **Class 0-3 vs. 4** | | | | |
| First risk | 1.56 | 0.247 | 0.73 | 3.32 |
| First protective | 0.87 | 0.727 | 0.40 | 1.90 |

**Table 14. Order of the infections (conditional logistic regression analysis), compared to class 0. Middle criteria approach.**

| **Order** | **Odds ratio** | **P-value** | **95% Conf. Interval** | |
|---|---|---|---|---|
| **Class 0-3 vs. 4** | | | | |
| First risk | **3.31** | **<0.001** | **1.83** | **5.99** |
| First protective | **2.10** | **0.025** | **1.10** | **4.00** |

All these three analyses showed trends supporting especially the hypothesis of CBV1's risk character but also the hypothesis of protective viruses. In the second type of analysis the classes 0 and 2 were combined and class 1 was analyzed against this new combined class. In this analysis the risk effect of CBV1 becomes stronger (Table 15).

**Table 15. CBV1 infections first (conditional logistic regression analysis), compared to combined classes 0 and 2. Tight criteria approach.**

| **Order** | **Odds ratio** | **P-value** | **95% Conf. Interval** | |
|---|---|---|---|---|
| First risk | 1.40 | 0.151 | 0.88 | 2.22 |

To study the protective effect more closely the risk class (class 1) was combined with the 0-class and this combined class was compared with class 2. In this analysis the protective effect gets stronger and the P-value comes very close to statistical significance (Table 16).

**Table 16. Protective infections first (conditional logistic regression analysis), compared to combined classes 0 and 1. Tight criteria approach.**

| **Order** | **Odds ratio** | **P-value** | **95% Conf. Interval** | |
|---|---|---|---|---|
| First protective | **0.55** | **0.060** | 0.29 | 1.03 |

In the following analysis the protective viruses CBV3 and CBV6 have been handled as separate cases and the analyses were done using both tight, middle and loose criteria approaches (Tables 17, 18 and 19).

**Table 17. Order of the infections (conditional logistic regression analysis), compared to class 0. CBV3 vs. CBV1 and CBV6 vs. CBV1 handled as separate groups. Tight criteria approach.**

| **CBV3 vs. CBV1** | **Odds ratio** | **P-value** | **95% Conf. Interval** | |
|---|---|---|---|---|
| CBV1 first | 1.25 | 0.338 | 0.79 | 1.95 |
| CBV3 first | 0.41 | 0.104 | 0.14 | 1.20 |

| **CBV6 vs. CBV1** | | | | |
|---|---|---|---|---|
| CBV1 first | 1.38 | 0.170 | 0.87 | 2.17 |
| CBV6 first | 0.62 | 0.236 | 0.29 | 1.36 |

**Table 18. Order of the infections (conditional logistic regression analysis), compared to class 0. CBV3 vs. CBV1 and CBV6 vs. CBV1 handled as separate groups. Middle criteria approach.**

| **Class 0-3 vs. 4** | **Odds ratio** | **P-value** | **95% Conf. Interval** | |
|---|---|---|---|---|
| **CBV3 vs. CBV1** | | | | |
| CBV1 first | **2.76** | **<0.001** | **1.75** | **4.34** |
| CBV3 first | 1.30 | 0.706 | 0.33 | 5.05 |

| **CBV6 vs. CBV1** | | | | |
|---|---|---|---|---|
| CBV1 first | **3.67** | **<0.001** | **2.00** | **6.72** |
| CBV6 first | **2.48** | **0.007** | **1.28** | **4.80** |

**Table 19. Order of the infections (conditional logistic regression analysis), compared to class 0. CBV3 vs. CBV1 and CBV6 vs. CBV1 handled as separate groups. Loose criteria approach.**

| **Class 0-3 vs. 4** | **Odds ratio** | **P-value** | **95% Conf. Interval** | |
|---|---|---|---|---|
| **CBV3 vs. CBV1** | | | | |
| CBV1 first | 1.24 | 0.488 | 0.68 | 2.26 |
| CBV3 first | **0.41** | **0.057** | 0.16 | 1.03 |

| **CBV6 vs. CBV1** | | | | |
|---|---|---|---|---|
| CBV1 first | **2.37** | **0.072** | 0.92 | 6.06 |
| CBV6 first | 1.85 | 0.217 | 0.70 | 4.91 |

According to tight criteria analyses the trends were as expected showing risk trend for CBV1 and protective trends for CBV3 and CBV6. In the middle criteria analysis, once again, the risk character of CBV1 was clearly evidenced. In the loose criteria analyses the protective effect of CBV3 clearly stands out and in CBV6 vs. CBV1 comparison the risk effect of CBV1 is obvious.

In conclusion, the chronology studies provided supportive data for the risk character of CBV1 and the protective character especially for CBV3 and to a lesser extent for CBV6, too. Based on these findings it can be concluded that the chronology of these infections influence the risk effect of CBV1. High risk is associated with CBV1 infection without prior exposure to CBV3 or CBV6. On the other hand, the risk effect is attenuated by previous exposure to CBV3 or CBV6.

### Conclusions

When all analyses are combined, we can conclude that a statistically significant association between enteroviruses and disease process leading to type 1 diabetes was found. Enteroviruses can have a risk effect (i.e. induce the diabetic disease process) or a protective effect. These findings can be utilized in diagnostic assays.

The most interesting results are related to the CBV serotypes. In this subgroup the strongest single discovery was the definite risk character of CBV1. It was first identified in relation to the time point of seroconversion to autoantibody positivity by seroneutralization analyses showing that CBV1 infections precede the initial autoantibody seroconversion. Later this result was confirmed by proving that the CBV1 infections hit more often the case children as compared to controls in the time window which immediately precedes autoantibody seroconversion. On the other hand, strong protective properties were associated with CBV serotypes CBV3 and CBV6. Also these associations were first identified by seroneutralization analyses in relation to the time of seroconversion to autoantibody positivity. The timing and chronology studies supported specific interactions between CBV1, CBV3 and CBV6 where the diabetogenic effect of CBV1 is attenuated by prior exposure to either CBV3 or CBV6 or both.

### Multi-time point seroneutralization analysis further including CBV2, CBV4 and CBV5

In this analysis also the remaining three CBV serotypes were analyzed in similar multi-time-point setting as CBV1, CBV3 and CBV6.

### Timing of infections

To clarify the possible timing of CBV subgroup infections, the same time points were selected for the seroneutralization analyses as described above.

The results presented below are based on the seroneutralization analysis of about 2000 samples for each of these CBV viruses. In order to study the timing of infections caused by the six CBV serotypes the recognized infections were categorized to different time windows relative to the date of seroconversion to autoantibody positivity (AAB+ date). The used windows were numbered from 0 to 3 as described above.

The same loose, middle and tight criteria approaches were used as described above. Statistical analyses were done using conditional logistic regression. The results of statistical analyses for the six CBV serotypes are shown in Tables 20-25.

**Table 20. CBV1 infection timing analysis. Loose criteria approach with classes 0-3 vs. 4.**

| **CBV1** | **Odds ratio** | **P-value** | **95% Conf. Interval** | |
|---|---|---|---|---|
| **Class 0-3 vs. 4** | | | | |
| Over 12 months before AAB+ date | **1.89** | **0.035** | **1.05** | **3.40** |
| 12-6 months before AAB+ date | **2.27** | **0.005** | **1.28** | **4.03** |
| 6-0 months before AAB+ date | **3.54** | **<0.001** | **1.95** | **6.42** |

**Table 21. CBV2 infection timing analysis. Loose, Middle and Tight criteria approaches with classes 0-3 vs. 4.**

| **CBV2** | **Odds ratio** | **P-value** | **95% Conf. Interval** | |
|---|---|---|---|---|
| **Loose criteria** | | | | |
| Over 12 months before AAB+ date | 0.79 | 0.441 | 0.44 | 1.43 |
| 12-6 months before AAB+ date | 1.38 | 0.236 | 0.81 | 2.34 |
| 6-0 months before AAB+ date | **2.32** | **0.001** | **1.40** | **3.84** |

| **Middle criteria** | | | | |
|---|---|---|---|---|
| Over 12 months before AAB+ date | 0.80 | 0.523 | 0.41 | 1.58 |
| 12-6 months before AAB+ date | 1.02 | 0.946 | 0.54 | 1.93 |
| 6-0 months before AAB+ date | **2.29** | **0.001** | **1.43** | **3.66** |

| **Tight Criteria** | | | | |
|---|---|---|---|---|
| Over 12 months before AAB+ date | 0.88 | 0.838 | 0.26 | 3.00 |
| 12-6 months before AAB+ date | 1.00 | 0.994 | 0.33 | 3.03 |
| 6-0 months before AAB+ date | **2.44** | **0.059** | **0.97** | **6.15** |

**Table 22. CBV3 infection timing analysis. Loose criteria approach with classes 0-3 vs. 4.**

| **CBV3** | **Odds ratio** | **P-value** | **95% Conf. Interval** | |
|---|---|---|---|---|
| **Class 0-3 vs. 4** | | | | |
| Over 12 months before AAB+ date | 0.42 | 0.058 | 0.18 | 1.03 |
| 12-6 months before AAB+ date | 0.84 | 0.609 | 0.43 | 1.63 |
| 6-0 months before AAB+ date | 0.69 | 0.331 | 0.33 | 1.45 |

**Table 23. CBV4 infection timing analysis. Loose, Middle and Tight criteria approaches with classes 0-3 vs. 4.**

| **CBV4** | **Odds ratio** | **P-value** | **95% Conf. Interval** | |
|---|---|---|---|---|
| **Loose criteria** | | | | |
| Over 12 months before AAB+ date | 0.51 | 0.135 | 0.21 | 1.24 |
| 12-6 months before AAB+ date | 1.05 | 0.887 | 0.53 | 2.09 |
| 6-0 months before AAB+ date | 1.07 | 0.868 | 0.48 | 2.41 |

| **Middle criteria** | | | | |
|---|---|---|---|---|
| Over 12 months before AAB+ date | 0.50 | 0.380 | 0.11 | 2.35 |
| 12-6 months before AAB+ date | -* | -* | -* | -* |
| 6-0 months before AAB+ date | 0.45 | 0.363 | 0.08 | 2.50 |

| **Tight Criteria** | | | | |
|---|---|---|---|---|
| Over 12 months before AAB+ date | 1.00 | 0.999 | 0.09 | 11.04 |
| 12-6 months before AAB+ date | -* | -* | -* | -* |
| 6-0 months before AAB+ date | 0.59 | 0.685 | 0.05 | 7.43 |

| | | | | |
|---|---|---|---|---|
| *Due to the low overall frequency of CBV4 infections there were no CBV4 infections in cases in 12-6 months window according to middle and tight criteria approaches. | | | | |

**Table 24. CBV5 infection timing analysis. Loose, Middle and Tight criteria approaches with classes 0-3 vs. 4.**

| **CBV5** | **Odds ratio** | **P-value** | **95% Conf. Interval** | |
|---|---|---|---|---|
| **Loose criteria** | | | | |
| Over 12 months before AAB+ date | 0.56 | 0.209 | 0.23 | 1.38 |
| 12-6 months before AAB+ date | 1.11 | 0.755 | 0.57 | 2.19 |
| 6-0 months before AAB+ date | 1.60 | 0.211 | 0.76 | 3.37 |

| **Middle criteria** | | | | |
|---|---|---|---|---|
| Over 12 months before AAB+ date | -* | -* | -* | -* |
| 12-6 months before AAB+ date | 0.88 | 0.849 | 0.23 | 3.35 |
| 6-0 months before AAB+ date | 1.45 | 0.488 | 0.51 | 4.14 |

| **Tight Criteria** | | | | |
|---|---|---|---|---|
| Over 12 months before AAB+ date | -§ | -§ | -§ | -§ |
| 12-6 months before AAB+ date | -§ | -§ | -§ | -§ |
| 6-0 months before AAB+ date | -§ | -§ | -§ | -§ |

| | | | | |
|---|---|---|---|---|
| *There were no CBV5 infections in cases in over 12 months window according to middle criteria approach. §In tight criteria approach the number of the observations decreased so low that no statistically analyzed results could be generated. | | | | |

**Table 25. CBV6 infection timing analysis. Loose criteria approach with classes 0-3 vs.**

| **CBV6** | **Odds ratio** | **P-value** | **95% Conf. Interval** | |
|---|---|---|---|---|
| **Class 0-3 vs. 4** | | | | |
| Over 12 months before AAB+ date | 0.96 | 0.874 | 0.56 | 1.65 |
| 12-6 months before AAB+ date | 1.54 | 0.090 | 0.94 | 2.54 |
| 6-0 months before AAB+ date | 1.35 | 0.234 | 0.82 | 2.22 |

### Conclusions

In addition to CBV1 also the closely related CBV2 was found to be a risk virus in multi-time-point analyses especially in the 6-0 months before AAB+ time window.

PAR was also calculated for CBV2 using loose criteria OR=2.26 and the seroprevalence of 48% in background population, accordingly, we end up with PAR=37% (Figure 3).

Altogether, the results indicate that CBV2 is also a risk virus associated with the pathogenesis of T1D and therefore useful in diagnostics.

### Example 2. Stool analyses

Altogether, 4184 stool samples from 359 children (102 cases and 257 controls) have been screened for enterovirus (EV) positivity. In Table 26 all these samples mentioned above have been divided into different categories. In EV screening PCR (J Med Virol. 1999 Nov;59(3):378-84. Diagnosis of enterovirus and rhinovirus infections by RT-PCR and time-resolved fluorometry with lanthanide chelate labeled probes. Lönnrot M, Sjöroos M, Salminen K, Maaronen M, Hyypiä T, Hyöty H.) in total 230 separate infections were identified out of which 166 could be serotyped by sequencing.

**Table 26. EV-screened stool samples divided into different categories.**

| | **Whole data** | **Case** | **Control** |
|---|---|---|---|
| Number of groups | 102 | | |
| Number of children | 359 | 102 | 257 |
| Number of stool samples | 4184 | 1321 | 2863 |
| Number of enterovirus positive stool samples | 307 | 103 | 204 |
| Amount of separate infections | 230 | 75 | 155 |
| Number of successfully serotyped infections | 166 (72%) | 54 (72%) | 112 (72%) |
| Number of untyped infections | 64 (28%) | 21 (28%) | 43 (28%) |
| Number of different serotypes | 28 | 17 | 24 |

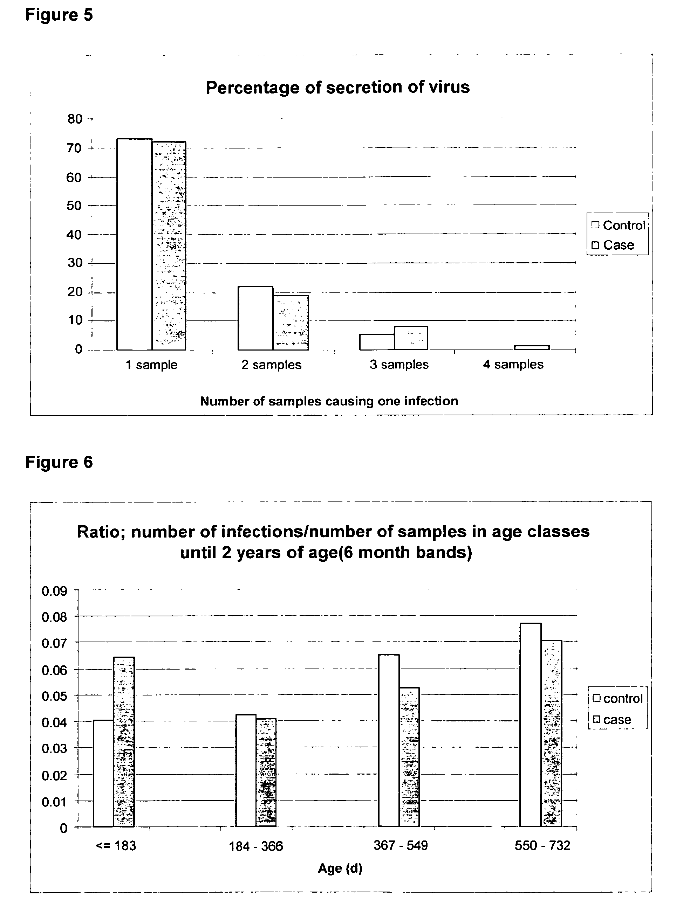

The duration of infections and the ratio of infections and samples in age classes are presented in Figures 5 and 6, respectively as follows: The duration of virus excretion is shown in Figure 5 as percentage of the number of serial samples positive for the same virus strain. The ratio of infections and samples in age classes (six months bands) until two years of age is shown in Figure 6. The ratio is calculated as number of infections in age class/number of all samples in the age class.

The strictly defined criteria were set for case and control children, according to which, for example, the difference in the day of birth between case child and control child was defined to be in maximum ± 2 months. In practice, in six case/control groups the difference of one control children was outside the defined criteria. However, of these in 5 cases the difference was only 64, 64, 65, 69 and 80 days making the deviation very small. Only in the case of one case/control group the deviation for one control child was marked the difference being 271 days. In addition, two control children turned type 1 diabetic during the follow up period.

### Serotyping the enteroviruses detected from stool samples in the whole cohort

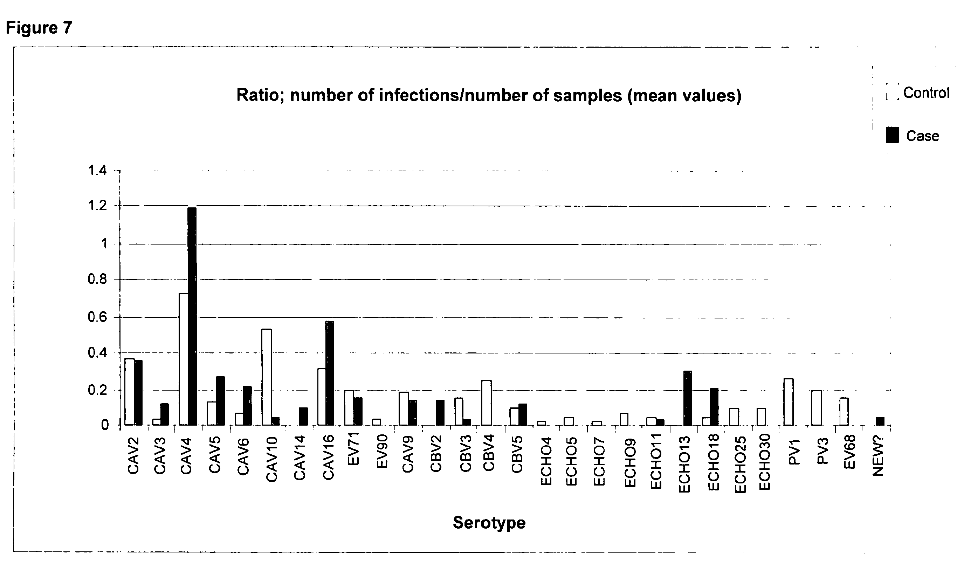

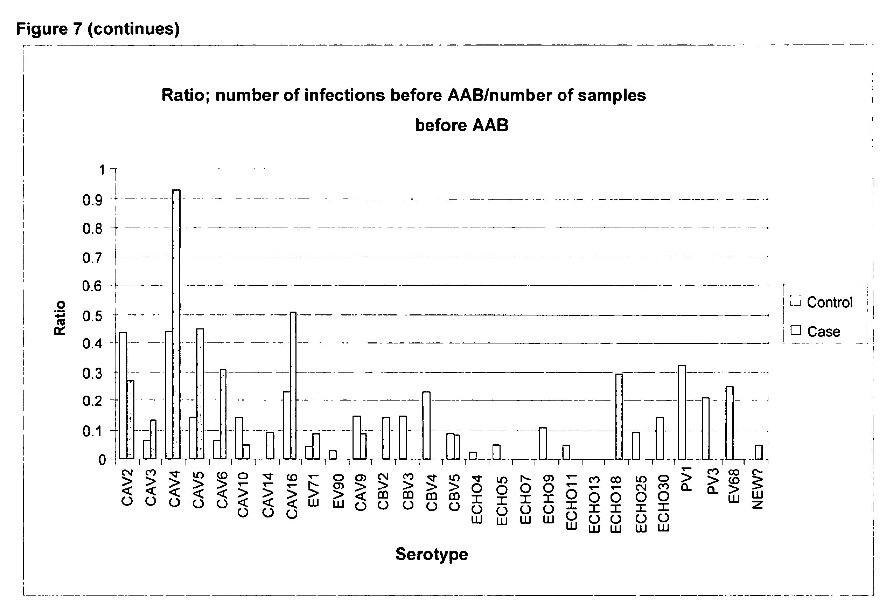

From all (307) enterovirus positive stool samples the serotype has been identified by VP1/VP2 (J Clin Microbiol. 2006 Aug;44(8):2698-704.Sensitive, seminested PCR amplification of VP1 sequences for direct identification of all enterovirus serotypes from original clinical specimens. Nasri D, Bouslama L, Omar S, Saoudin H, Bourlet T, Aouni M, Pozzetto B, Pillet S. Typing of human enterovirus by partial sequencing of VP2. J Clin Microbiol. 2007 Aug;45(8): 2370-9) sequencing from 166 infections (infection=same serotype in one or in two or more consecutive samples). Figure 7 shows the proportions of all identified serotypes presented as a ratio of infections and samples collected. In the upper panel the ratio of all the infections and samples is calculated as number of infections/number of samples, and in the lower panel the ratio of the infections and samples before the appearance of autoantibodies AAB is calculated as number of infections before AAB/number of samples before AAB. The ratio of different serotypes per samples collected is first calculated for every case child and every control child, here presented as mean value of all cases and all controls.

According to the above data, three kinds of serotypes can be seen. One group includes serotypes which are more common in case than in control children, another group comprises serotypes which are more frequent in control than in case children, and still another group those which are about equally common in both cases and controls. Based on this we formed the following risk groups of viruses: serotypes CAV4, CAV5, CAV6, CAV16 and ECHO 18 were chosen to be diabetogenic group 1; and serotypes CAV4, CAV5, CAV6, CBV2 and ECHO18 as diabetogenic group 2. When the frequency of infections by the risk group serotypes was analyzed as a group a statistically significant risk-effect was observed (Table 27). These analyses addressed the question if one or more infections by any of the risk group serotypes (vs. having none of these risk group serotypes) has a statistically significant effect.

High OR was received when comparing males and females separately. Before the appearance of autoantibodies, females had the highest OR (OR=6.48, P = 0.008). In contrast, males had the highest OR within the 12-month window before the before seroconversion to autoantibody positivity (OR=8.49, P = 0.008) (two risk groups combined). The highest OR was received one year before AAB in those children that had the risk HLA genotype (OR=9.86, P=0.004) (two risk groups combined). The same trend was also seen in diabetic cases and their controls; the highest ORs were received in a time period from birth to autoantibody seroconversion and one year before autoantibody seroconversion (Table 28).

**Table 27. Statistical analysis of different groups of serotypes; two risk groups, and two risk groups combined.**

| **case** | **Odds Ratio** | **P>\|z\|** | **95% lower limit** | **95% upper limit** |
|---|---|---|---|---|
| **Risk group 1** | | | | |
| **Whole cohort** | | | | |
| CAV4, CAV5, CAV6, CAV16, ECHO18 | **1.92** | **0.030** | **1.06** | **3.47** |
| **Whole cohort before AAB** | | | | |
| CAV4, CAV5, CAV6, CAV16, ECHO18 | **2.69** | **0.005** | **1.34** | **5.39** |
| **Whole cohort one year before AAB** | | | | |
| CAV4, CAV5, CAV6, CAV16, ECHO18 | **3.72** | **0.011** | **1.35** | **10.21** |

| **Risk group 2** | | | | |
|---|---|---|---|---|
| **Whole cohort** | | | | |
| CAV4, CAV5, CAV6, CBV2, ECHO18 | **2.39** | **0.008** | **1.25** | **4.57** |
| **Whole cohort before AAB** | | | | |
| CAV4, CAV5, CAV6, CBV2, ECHO18 | **3.16** | **0.003** | **1.46** | **6.84** |
| **Whole cohort one year before AAB** | | | | |
| CAV4, CAV5, CAV6, CBV2, ECHO18 | **6.53** | **0.001** | **2.07** | **20.60** |

| **Risk groups 1 and 2 combined** | | | | |
|---|---|---|---|---|
| **Whole cohort** | | | | |
| CAV4, CAV5, CAV6, CAV16, CBV2, ECHO18 | **1.92** | **0.03** | **1.06** | **3.47** |
| **Whole cohort before AAB** | | | | |
| CAV4, CAV5, CAV6, CAV16, CBV2, ECHO18 | **2.91** | **0.003** | **1.45** | **5.84** |
| **Whole cohort one year before AAB** | | | | |
| CAV4, CAV5, CAV6, CAV16, CBV2, ECHO18 | **7.30** | **0.001** | **2.32** | **22.89** |

**Table 28. Statistical analysis of different groups of serotypes; two risk groups. Only cases, which have T1 D and their controls.**

| **ONLY TYPE 1 DIABETES (T1D) CASES AND THEIR CONTROLS** | | | **102 cases** | **63 T1D cases*** |
|---|---|---|---|---|
| **Case** | **Odds Ratio** | **P>\|z\|** | **95% lower limit** | **95% upper limit** |
| **Risk group 1** | | | | |
| **Whole cohort** | | | | **4.85** |
| CAV4 CAV5, CAV6, CAV16, ECHO18 | **2.31** | **0.028** | **1.09** | |
| **Whole cohort before AAB** | | | | |
| CAV4 CAV5, CAV6, CAV16, ECHO18 | **4.49** | **0.003** | **1.67** | **12.07** |
| **Whole cohort one year before AAB** | | | | |
| CAV4 CAV5, CAV6, CAV16, ECHO18 | **3.99** | **0.029** | **1.14** | **13.87** |

| **Risk group 2** | | | | |
|---|---|---|---|---|
| **Whole cohort** | | | | |
| CAV4 CAV5, CAV6, CBV2, ECHO18 | **2.91** | **0.014** | **1.24** | **6.84** |
| **Whole cohort before AAB** | | | | |
| CAV4 CAV5, CAV6, CBV2, ECHO18 | **5.58** | **0.004** | **1.73** | **18.05** |
| **Whole cohort one year before AAB** | | | | |
| CAV4 CAV5, CAV6, CBV2, ECHO18 | **5.04** | **0.02** | **1.28** | **19.78** |

| | | | | |
|---|---|---|---|---|
| *Only the cases which progressed to clinical T1D during prospective observation, and their controls | | | | |

The number of children and identified infections in combined risk groups 1 and 2 are presented in Table 29.

**Table 29. Number of children and infections in combined risk groups 1 and 2.**

| | | **Number of children** | | **Number of infections** | |
|---|---|---|---|---|---|
| | | **Case** | **Control** | **Case** | **Control** |
| **Risk groups 1 and 2 combined** | **Whole cohort** | **25** | **39** | **33** | **45** |
| | **Before AAB** | **21** | **21** | **25** | **29** |
| | **Year Before AAB** | **13** | **6** | **13** | **13** |

It seems that in the stool analysis the risk effect of certain coxsackie A viruses is seen if the infection is suffered in very young age and especially if child has the moderate risk HLA genotype. In addition they might be associated with an aggressive autoimmune process which leads to severe beta cell damage and diabetes.

In conclusion, when several serotypes were combined, a group of risk viruses could be recognized. CAV4, CAV5, CAV6, CAV16 and E18 are the most interesting as potential risk viruses. It is remarkable that CAV4, CAV5, CAV6 and CAV16 are genetically closely related to each other. The clearest risk effect was associated with male gender in the restricted time window before the autoantibody seroconversion date.

### Summary of serum and stool analyzes

The following serotype scoring scheme was created showing the most promising diabetogenic and protective viruses. The analysis type suggesting different character and the strength of the corresponding result are also shown in scale (+)/+/++/+++. All these analyses were done from single time point representing the first autoantibody positive sample.

**Example 3. Material showing the protective character of CBV4 and CBV5, and supporting protective character of CBV3 and risk character of CBV2**

| **Virus** | | **Property** | **Analysis type** | |
|---|---|---|---|---|
| | **Diabetogenic** | **Protective** | **Seroneutralization** | **Stool samples** |
| **CBV1** | **X** | | **+++** | |
| **CBV2** | **X** | | **++** | |
| **Echo32** | **X** | | **++** | |
| **Echo29** | **X** | | **+** | |
| **Echo14** | **X** | | **+** | |
| **CAV4** | **X** | | | **+** |
| **CAV16** | **X** | | | **+** |
| **CAV5** | **X** | | | **+** |
| **CAV6** | **X** | | | **(+)** |
| **Echo18** | **X** | | | **(+)** |
| **Echo3** | **X** | | **(+)** | |
| **CBV3** | | **X** | **+++** | **++** |
| **CBV6** | | **X** | **++** | |
| **CBV4** | | **X** | **++** | **+** |
| **CBV5** | | **X** | **++** | |
| **Echo11** | | **X** | **+** | **+** |
| **Echo13** | | **X** | **(+)** | **(+)** |

### Analysis of samples taken at the age of one year

In this study altogether 284 serum samples were collected from children at the age of one year (range 321 - 430 days) and their seroneutralization responses were studied with six CBV serotypes. When analyzed statistically using paired case-control design, CBV3 and especially CBV4 were found to be protective (Table 30). Infections at young age can thus be expected to provide protection against an early starting diabetogenic autoimmune process.

**Table 30. Conditional logistic regression analysis of 6 CBV serotypes in the samples taken at the age of one year. Loose criteria approach: classes 0-3 vs. 4.**

| **OR** | **P** | **95% Conf. Interval** | |
|---|---|---|---|
| **CBV1** | | | |
| 1.05 | 0.857 | 0.62 | 1.79 |

| **CBV2** | | | |
|---|---|---|---|
| 1.04 | 0.89 | 0.6 | 1.79 |

| **CBV3** | | | |
|---|---|---|---|
| 0.35 | 0.019 | 0.15 | 0.84 |

| **CBV4** | | | |
|---|---|---|---|
| 0.16 | 0.001 | 0.06 | 0.48 |

| **CBV5** | | | |
|---|---|---|---|
| 1.2 | 0.724 | 0.43 | 3.34 |

| **CBV6** | | | |
|---|---|---|---|
| 1.01 | 0.961 | 0.57 | 1.8 |

### Time point of type 1 diabetes diagnosis

Altogether, 215 serum samples were available from the children progressed to type 1 diabetes and their corresponding controls. These samples were analyzed by carrying out seroneutralization analyses with six CBV viruses. When statistical analyses were done for these viruses with cross-sectional setup, CBV5 showed a trend for a protective character (Table 31). This result suggests that CBV5 has an attenuating effect in the type 1 diabetes autoimmunity process after this process has already started.

**Table 31. Conditional logistic regression analysis of 6 CBV serotypes in samples taken at the time point of type 1 diabetes diagnosis. Loose criteria approach: classes 0-3 vs. 4.**

| **OR** | **P** | **95% Conf.Interval** | |
|---|---|---|---|
| **CBV1** | | | |
| 0.98 | 0.961 | 0.56 | 1.74 |

| **CBV2** | | | |
|---|---|---|---|
| 1.38 | 0.311 | 0.74 | 2.55 |

| **CBV3** | | | |
|---|---|---|---|
| 0.68 | 0.489 | 0.23 | 2 |

| **CBV4** | | | |
|---|---|---|---|
| 0.94 | 0.917 | 0.27 | 3.24 |

| **CBV5** | | | |
|---|---|---|---|
| 0.34 | 0.095 | 0.1 | 1.2 |

| **CBV6** | | | |
|---|---|---|---|
| 0.92 | 0.803 | 0.48 | 1.77 |

### Sample interval between autoantibody seroconversion date and diagnosis of T1D

Seroneutralization results of autoantibody seroconversion date done with six CBV serotypes were compared to results of the samples from type 1 diabetes diagnosis date taken from the same children and using the same viruses. In this analysis the reference group consisted of children who were negative for a given virus in both samples. Also in this analysis a protective trend for CBV5 was detected suggesting attenuating of the type 1 diabetes autoimmunity process (Table 32). Interestingly, CBV2 showed a risk trend with high OR indicating that it may accelerate the autoimmune process progression after its initiation.

**Table 32. Conditional logistic regression analysis of 6 CBV serotypes. Sample interval between autoantibody seroconversion date and type 1 diabetes diagnosis date were compared. Loose criteria approach: classes 0-3 vs. 4. Changes from negative to positive have been analyzed against reference group negative to negative.**

| **OR** | **P** | **95% Conf.Interval** | |
|---|---|---|---|
| **CBV1** | | | |
| 0.82 | 0.797 | 0.18 | 3.78 |

| **CBV2** | | | |
|---|---|---|---|
| 6.04 | 0.106 | 0.68 | 53.32 |

| **CBV3** | | | |
|---|---|---|---|
| 0.78 | 0.778 | 0.14 | 4.36 |

| **CBV4** | | | |
|---|---|---|---|
| 0.35 | 0.339 | 0.04 | 3.02 |

| **CBV5** | | | |
|---|---|---|---|
| 0.18 | 0.108 | 0.023 | 1.45 |

| **CBV6** | | | |
|---|---|---|---|
| 0.89 | 0.813 | 0.35 | 2.27 |

### Conclusion

The analysis of the samples taken at the age of one year showed that CBV3 and CBV4 infections protect children from type 1 diabetes. The results suggest that early occurring CBV3 and CBV4 infections may give a protection against early starting diabetogenic autoimmune process. In addition, a protective trend could also be associated for CBV5 when samples taken at the type 1 diabetes diagnosis date were analyzed and when the time interval between autoantibody seroconversion and type 1 diabetes diagnosis date were compared. These results suggest that the mechanism of the protection for the CBV4 infections may be the attenuation of the ongoing type 1 diabetes autoimmune process. Furthermore, the link between CBV2 infections and risk of type 1 diabetes was detected when the data of interval between autoantibody seroconversion date samples and the samples taken at the date of diabetes diagnosis were compared.

## Claims

1. A diagnostic assay for assessing the risk of type 1 diabetes (T1 D) comprising determining the presence of at least one risk or at least one protective enterovirus serotype in a body sample, whereby
the presence of at least one risk serotype selected from the group consisting of CBV1, CBV2, Echo3, Echo14, Echo18, Echo29, Echo32, CAV4, CAV5, CAV6, and CAV16 indicates an increased risk of contracting T1D, or
the presence of at least one protective serotype selected from the group consisting of CBV3, CBV4, CBV5, CBV6, Echo11, and Echo13 indicates a reduced risk of contracting T1 D.

2. The diagnostic assay of claim 1, comprising determining the presence of at least one of CBV1, CBV2, CBV3, CBV4, CBV5 and CBV6.

3. The diagnostic assay of claim 1, comprising determining the presence of at least one of CBV1 and CBV2.

4. The diagnostic assay of claim 1, comprising determining the presence of at least one risk serotype, and at least one protective serotype.

5. The diagnostic assay of any one of claims 1 - 4, wherein the presence of the enterovirus is determined by detecting a component of the virus or an immune response thereto in the body sample.

6. The diagnostic assay of any one of claims 1 - 4, wherein the presence of the virus is determined by PCR, RT-PCR, *in situ* hybridization, immunohistochemistry, antigen detection using EIA or other methods, virus isolation, sequencing, electron microscopy, or immuno electron microscopy.

7. The diagnostic assay of any one of claims 1 - 4, wherein the presence of the virus is determined by detecting an immune response against the virus by measuring virus specific antibody response, or by measuring a cell-mediated immune response to the virus, or measurements of innate immune system activation.

8. A method of monitoring the efficacy of antiviral treatments aiming at prevention of T1D, comprising providing a sample from a subject having received antiviral treatment, and determining the presence of at least one risk or at least one protective enterovirus serotype in the sample, whereby
the presence of at least one risk serotype selected from the group consisting of CBV1, CBV2, Echo3, Echo14, Echo18, Echo29, Echo32, CAV4, CAV5, CAV6, and CAV16, indicates that the treatment has not reduced the risk of contracting T1D, or
the presence of at least one protective serotype selected from the group consisting of CBV3, CBV4, CBV5, CBV6, Echo11, and Echo13, indicates that the treatment has reduced the risk of contracting T1 D.

9. The method of claim 8, comprising determining the presence of at least one of CBV1, CBV2, CBV3, CBV4, CBV5 and CBV6.

10. The method of claim 8, comprising determining the presence of at least one of CBV1 and CBV2.

11. The method of claim 8, comprising determining the presence of at least one risk serotype and at least one protective serotype.

12. The method of any one of claims 8 - 11, wherein the presence of the enterovirus is determined by detecting a component of the virus or an immune response thereto in the body sample.

13. The method of any one of claims 8 - 11, wherein the presence of the virus is determined by PCR, RT-PCR, *in situ* hybridization, immunohistochemistry, antigen detection using EIA or other methods, virus isolation, sequencing, electron microscopy, or immuno electron microscopy.

14. The method of any one of claims 8 - 11, wherein the presence of the virus is determined by detecting an immune response against the virus by measuring virus specific antibody response, or by measuring a cell-mediated immune response to the virus, or measurements of innate immune system activation.

15. *In vitro* use of a diagnostic kit for assessing the risk of T1D or in monitoring the efficacy of antiviral treatments aiming at prevention of T1D, wherein the kit comprises means for determining the presence of at least one risk and at least one protective enterovirus serotype in a body sample, whereby the risk serotype is selected from the group consisting of CBV1, CBV2, Echo3, Echo14, Echo18, Echo29, Echo32, CAV4, CAV5, CAV6, and CAV16, and the protective serotype is selected from the group consisting of CBV3, CBV4, CBV5, CBV6, Echo11, and Echo13.

## Patentansprüche

1. Diagnostischer Assay zum Beurteilen des Risikos von Typ 1-Diabetes (T1D), umfassend das Bestimmen des Vorliegens mindestens eines Risikos oder mindestens eines schützenden Enterovirus-Serotyps in einer Körperprobe, wobei
das Vorliegen mindestens eines Risiko-Serotyps ausgewählt aus der Gruppe bestehend aus CBV1, CBV2, Echo3, Echo14, Echo18, Echo29, Echo32, CAV4, CAV5, CAV6 und CAV16 auf ein erhöhtes Risiko des Zuziehens von T1D hinweist oder
das Vorliegen mindestens eines schützenden Serotyps ausgewählt aus der Gruppe bestehend aus CBV3, CBV4, CBV5, CBV6, Echo11 und Echo13 auf ein reduziertes Risiko des Zuziehens von T1D hinweist.

2. Diagnostischer Assay nach Anspruch 1, umfassend das Bestimmen des Vorliegens von mindestens einem von CBV1, CBV2, CBV3, CBV4, CBV5 und CBV6.

3. Diagnostischer Assay nach Anspruch 1, umfassend das Bestimmen des Vorliegens von mindestens einem von CBV1 und CBV2.

4. Diagnostischer Assay auf nach Anspruch 1, umfassend das Bestimmen des Vorliegens von mindestens einem Risiko-Serotyp und mindestens einem schützenden Serotyp.

5. Diagnostischer Assay nach einem der Ansprüche 1-4, wobei das Vorliegen des Enterovirus durch Erfassen einer Komponente des Virus oder einer Immunantwort dazu in der Körperprobe bestimmt wird.

6. Diagnostischer Assay nach einem der Ansprüche 1-4, wobei das Vorliegen des Virus durch PCR, RT-PCR, In-situ-Hybridisierung, Immun-Histochemie, Antigenerfassung unter Anwendung von EIA oder anderen Verfahren, Virusisolierung, Sequenzieren, Elektronenmikroskopie oder Immunelektronenmikroskopie bestimmt wird.

7. Diagnostischer Assay nach einem der Ansprüche 1-4, wobei das Vorliegen des Virus durch Erfassen einer Immunantwort gegen das Virus durch Messen virusspezifischer Antikörperantwort oder durch Messen einer zellenvermittelten Immunantwort auf das Virus oder Messbestimmungen von angeborener Immunsystemaktivierung bestimmt wird.

8. Verfahren zum Überwachen der Wirksamkeit von Antivirusbehandlungen, die auf die Verhinderung von T1D hinzielen, umfassend das Bereitstellen einer Probe von einem Subjekt, das eine Antivirusbehandlung erhalten hat, und das Bestimmen des Vorliegens von mindestens einem Risiko oder mindestens einem schützenden Enterovirus-Serotyp in der Probe, wobei
das Vorliegen von mindestens einem Risiko-Serotyp ausgewählt aus der Gruppe bestehend aus CBV1, CBV2, Echo3, Echo14, Echo18, Echo29, Echo32, CAV4, CAV5, CAV6 und CAV16 darauf hinweist, dass die Behandlung das Risiko des Zuziehens von T1D nicht reduziert hat oder
das Vorliegen von mindestens einem schützenden Serotyp ausgewählt aus der Gruppe bestehend aus CBV3, CBV4, CBV5, CBV6, Echo11 und Echo13 darauf hinweist, dass die Behandlung das Risiko des Zuziehens von T1D reduziert hat.

9. Verfahren nach Anspruch 8, umfassend das Bestimmen des Vorliegens von mindestens einem von CBV1, CBV2, CBV3, CBV4, CBV5 und CBV6.

10. Verfahren nach Anspruch 8, umfassend das Bestimmen des Vorliegens von mindestens einem von CBV1 und CBV2.

11. Verfahren nach Anspruch 8, umfassend das Bestimmen des Vorliegens von mindestens einem Risiko-Serotyp und mindestens einem schützenden Serotyp.

12. Verfahren nach einem der Ansprüche 8-11, wobei das Vorliegen des Enterovirus durch Erfassen einer Komponente des Virus oder einer Immunantwort dazu in der Körperprobe bestimmt wird.

13. Verfahren nach einem der Ansprüche 8-11, wobei das Vorliegen des Virus durch PCR, RT-PCR, In-situ-Hybridisierung, Immunhistochemie, Antigenerfassung unter Anwendung von EIA oder anderen Verfahren, Virusisolierung, Sequenzieren, Elektronenmikroskopie oder Immunelektronenmikroskopie bestimmt wird.

14. Verfahren nach einem der Ansprüche 8-11, wobei das Vorliegen des Virus durch Erfassen einer Immunantwort gegen das Virus durch Messen einer virusspezifischen Antikörperantwort oder durch Messen einer zellenvermittelten Immunantwort auf das Virus oder Messbestimmungen von angeborener Immunsystemaktivierung bestimmt wird.

15. In-Vitro-Verwendung eines diagnostischen Kits zum Beurteilen des Risikos von T1D oder beim Kontrollieren der Wirksamkeit von Antivirusbehandlungen, die auf die Verhinderung von T1D hinzielen, wobei das Kit Folgendes umfasst
ein Mittel zum Bestimmen des Vorliegens von mindestens einem Risiko und mindestens einem schützenden Enterovirus-Serotyp in einer Körperprobe, wobei der Risiko-Serotyp aus der Gruppe ausgewählt ist bestehend aus CBV1, CBV2, Echo3, Echo14, Echo18, Echo29, Echo32, CAV4, CAV5, CAV6 und CAV16 und der schützende Serotyp ausgewählt ist aus der Gruppe bestehend aus CBV3, CBV4, CBV5, CBV6, Echo11, und Echo13.

## Revendications

1. Essai diagnostique pour évaluer le risque de diabète de type 1 (DT1) comprenant la détermination de la présence d'au moins un sérotype d'entérovirus à risque ou d'au moins un sérotype d'entérovirus protecteur dans un échantillon corporel, moyennant quoi
la présence d'au moins un sérotype à risque sélectionné dans le groupe consistant en CBV1, CBV2, Echo3, Echo14, Echo18, Echo29, Echo32, CAV4, CAV5, CAV6, et CAV16 indique un risque accru de contracter le DT1, ou
la présence d'au moins un sérotype protecteur sélectionné dans le groupe consistant en CBV3, CBV4, CBV5, CBV6, Echo11, et Echo13 indique un risque réduit de contracter le DT1.

2. Essai diagnostique selon la revendication 1, comprenant la détermination de la présence d'au moins l'un de CBV1, CBV2, CBV3, CBV4, CBV5 et CBV6.

3. Essai diagnostique selon la revendication 1, comprenant la détermination de la présence d'au moins l'un de CBV1 et CBV2.

4. Essai diagnostique selon la revendication 1, comprenant la détermination de la présence d'au moins un sérotype à risque, et d'au moins un sérotype protecteur.

5. Essai diagnostique selon l'une quelconque des revendications 1 à 4, dans lequel la présence de l'entérovirus est déterminée par la détection d'un composant du virus ou d'une réponse immunitaire à celui-ci dans l'échantillon corporel.

6. Essai diagnostique selon l'une quelconque des revendications 1 à 4, dans lequel la présence du virus est déterminée par PCR, RT-PCR, hybridation *in situ,* immunohistochimie, une détection d'antigène en utilisant un EIA ou d'autres procédés, un isolement du virus, un séquençage, la microscopie électronique, ou la microscopie immunoélectronique.

7. Essai diagnostique selon l'une quelconque des revendications 1 à 4, dans lequel la présence du virus est déterminée par la détection d'une réponse immunitaire contre le virus en mesurant la réponse d'anticorps spécifiques au virus, ou en mesurant une réponse immunitaire à médiation cellulaire contre le virus, ou par des mesures de l'activation du système immunitaire inné.

8. Procédé pour surveiller l'efficacité de traitements antiviraux visant à la prévention du DT1, comprenant la mise à disposition d'un échantillon d'un sujet ayant reçu un traitement antiviral, et la détermination de la présence d'au moins un sérotype d'entérovirus à risque ou d'au moins un sérotype d'entérovirus protecteur dans l'échantillon, moyennant quoi
la présence d'au moins un sérotype à risque sélectionné dans le groupe consistant en CBV1, CBV2, Echo3, Echo14, Echo18, Echo29, Echo32, CAV4, CAV5, CAV6, et CAV16, indique que le traitement n'a pas réduit le risque de contracter le DT1, ou
la présence d'au moins un sérotype protecteur sélectionné dans le groupe consistant en CBV3, CBV4, CBV5, CBV6, Echo11, et Echo13, indique que le traitement a réduit le risque de contracter le DT1.

9. Procédé selon la revendication 8, comprenant la détermination de la présence d'au moins l'un de CBV1, CBV2, CBV3, CBV4, CBV5 et CBV6.

10. Procédé selon la revendication 8, comprenant la détermination de la présence d'au moins l'un de CBV1 et CBV2.

11. Procédé selon la revendication 8, comprenant la détermination de la présence d'au moins un sérotype à risque et d'au moins un sérotype protecteur.

12. Procédé selon l'une quelconque des revendications 8 à 11, dans lequel la présence de l'entérovirus est déterminée par la détection d'un composant du virus ou d'une réponse immunitaire à celui-ci dans l'échantillon corporel.

13. Procédé selon l'une quelconque des revendications 8 à 11, dans lequel la présence du virus est déterminée par PCR, RT-PCR, hybridation *in situ,* immunohistochimie, une détection d'antigène en utilisant un EIA ou d'autres procédés, un isolement du virus, un séquençage, la microscopie électronique, ou la microscopie immunoélectronique.

14. Procédé selon l'une quelconque des revendications 8 à 11, dans lequel la présence du virus est déterminée par la détection d'une réponse immunitaire contre le virus en mesurant la réponse d'anticorps spécifiques au virus, ou en mesurant une réponse immunitaire à médiation cellulaire contre le virus, ou par des mesures de l'activation du système immunitaire inné.

15. Utilisation *in vitro* d'un kit de diagnostic pour évaluer le risque de DT1 ou dans la surveillance de l'efficacité de traitements antiviraux visant à la prévention du DT1, dans laquelle le kit comprend des moyens pour déterminer la présence d'au moins un sérotype d'entérovirus à risque et d'au moins un sérotype d'entérovirus protecteur dans un échantillon corporel, moyennant quoi le sérotype à risque est sélectionné dans le groupe consistant en CBV1, CBV2, Echo3, Echo14, Echo18, Echo29, Echo32, CAV4, CAV5, CAV6, et CAV16, et le sérotype protecteur est sélectionné dans le groupe consistant en CBV3, CBV4, CBV5, CBV6, Echo11, et Echo13.
